# EUROPEAN PATENT SPECIFICATION

(11) **EP 3 586 833 B1**
(45) Date of publication and mention of the grant of the patent: **07.07.2021**
(21) Application number: 18756570.0
(22) Date of filing: 15.02.2018
(51) Int. Cl.: A61K 9/70, A61K 47/32

(54) **TRANSDERMAL PATCH BASE AND TRANSDERMAL PATCH USING SAME**
TRÄGER FÜR TRANSDERMALES PFLASTER UND TRANSDERMALES PFLASTER DAMIT
BASE DE PATCH TRANSDERMIQUE ET PATCH TRANSDERMIQUE L'UTILISANT

(30) Priority: 21.02.2017 JP 2017029841
(43) Date of publication of application: 01.01.2020
(73) Proprietor: Hisamitsu Pharmaceutical Co., Inc., Tosu-shi, Saga 841-0017 (JP)
(72) Inventor: TSURUSHIMA, Keiichiro, Tosu-shi Saga 841-0017 (JP); SAEKI, Masakazu, Tosu-shi Saga 841-0017 (JP); YOSHINAGA, Takaaki, Tosu-shi Saga 841-0017 (JP)
(74) Representative: Grünecker Patent- und Rechtsanwälte PartG mbB
(86) International application number: PCT/JP2018/005337
(87) International publication number: WO 2018/155310

(56) References cited:
- WO-A1-2006/090782
- WO-A1-2015/025935
- WO-A1-2016/104226
- WO-A1-2016/104227
- WO-A1-2016/136556
- JP-A- H09 217 083
- US-A1- 2016 175 262
- US-A1- 2016 206 569
- US-B1- 6 407 293

## Description

### [Technical Field]

The present invention relates to a patch base and a patch using the same, and more specifically relates to a patch base containing gelatin and polyvinyl alcohol, and a patch using the same.

### [Background Art]

A patch base contains a water soluble polymer such as gelatin, polyacrylic acid, polyacrylate, or polyvinyl alcohol in order for a base layer to have a high moisture content and ensure excellent shape retention and cohesiveness. In addition, the patch base also contains a cooling agent such as L-menthol in order to exhibit a cooling effect (for example, International Publication No. WO2006/090782 (PTL 1), International Publication No. WO2015/025935 (PTL 2) and US Patent Application Publication No. 2016/0175262 (PTL 4)).

However, in few cases, the conventional patch base is considered to be problematic due to a strong irritating odor of L-menthol used as a cooling agent. In this regard, as an odorless cooling agent alternative to L-menthol, L-menthyl glyceryl ether has been known and derivatives thereof have also been proposed (for example, Japanese Unexamined Patent Application Publication No. Hei 9-217083 (PTL 3)) .

### [Citation List]

### [Patent Literature]

[PTL 1] International Publication No. WO2006/090782
[PTL 2] International Publication No. WO2015/025935
[PTL 3] Japanese Unexamined Patent Application Publication No. Hei 9-217083
[PTL 4] US Patent Application Publication No. 2016/0175262

### [Summary of Invention]

### [Technical Problem]

The present inventors, however, have found that the conventional patch base still generates an unpleasant odor even after the cooling agent is changed over from L-menthol to L-menthyl glyceryl ether.

The present invention has been made in view of the above problem, and has an object to provide a patch base and a patch using the same, which retain an adhesive force of a base and inhibits generation of an unpleasant odor even in the case of using L-menthyl glyceryl ether as a cooling agent.

### [Solution to Problem]

The present inventors have continuously conducted earnest studies to achieve the above object, and consequently have found that causes for generation of an unpleasant odor from a conventional patch base containing L-menthyl glyceryl ether as a cooling agent are gelatin and polyvinyl alcohol contained in the base, and further found that when a poly(methyl acrylate/2-ethylhexyl acrylate) copolymer is blended into the base, an unpleasant odor generated from gelatin and polyvinyl alcohol is inhibited. Thus, the present inventors have completed the present invention.

Specifically, a patch base of the present invention is a base comprising gelatin, polyvinyl alcohol, a methyl acrylate/2-ethylhexyl acrylate copolymer, and L-menthyl glyceryl ether, wherein
relative to the total mass of the base,
the content of the gelatin is 1.5 to 6% by mass,
the content of the polyvinyl alcohol is 0.5 to 8% by mass, and
the content of the methyl acrylate/2-ethylhexyl acrylate copolymer is 0.5 to 13% by mass.

The patch base of the present invention preferably further contains at least one adhesive agent selected from the group consisting of polyacrylic acids and polyacrylic acid neutralized products.

Moreover, a patch of the present invention comprises an adhesive layer containing the base of the present invention and a drug on a backing.

### [Advantageous Effects of Invention]

According to the present invention, it is possible to provide a patch base and a patch using the same, which retain an adhesive force of the base and inhibit generation of an unpleasant odor even in the case of using L-menthyl glyceryl ether as a cooling agent.

### [Description of Embodiments]

Hereinafter, the present invention will be described in detail based on preferred embodiments thereof.

First, a patch base of the present invention is elaborated on. The patch base of the present invention is a base comprising gelatin, polyvinyl alcohol, a methyl acrylate/2-ethylhexyl acrylate copolymer, and L-menthyl glyceryl ether, wherein
relative to the total mass of the base,
the content of the gelatin is 1.5 to 6% by mass,
the content of the polyvinyl alcohol is 0.5 to 8% by mass, and
the content of the methyl acrylate/2-ethylhexyl acrylate copolymer is 0.5 to 13% by mass.

The gelatin used in the present invention is not particularly limited, but may be any gelatin generally used for patches. In the patch base of the present invention, the content of the gelatin is 1.5 to 6% by mass relative to the total mass of the base. A base only containing less than 1.5% by mass of gelatin does not generate an unpleasant odor originated from the gelatin, even though the methyl acrylate/2-ethylhexyl acrylate copolymer is not blended to the base. For this reason, bases each containing 1.5% by mass or more (preferably 2% by mass or more) of gelatin need to be targeted for the case where the methyl acrylate/2-ethylhexyl acrylate copolymer is blended in the base for the purpose of inhibiting generation of an unpleasant odor originated from the gelatin. On the other hand, if the content of the gelatin exceeds 6% by mass, the adhesive force of the base decreases. From the viewpoint of ensuring a sufficient adhesive force of the base, the upper limit of the content of the gelatin is preferably 5% by mass or less, and more preferably 4% by mass or less.

The polyvinyl alcohol used in the present invention is not particularly limited, but may be any polyvinyl alcohol generally used for patches. In the patch base of the present invention, the content of the polyvinyl alcohol is 0.5 to 8% by mass relative to the total mass of the base. A base only containing less than 0.5% by mass of polyvinyl alcohol does not generate an unpleasant odor originated from the polyvinyl alcohol, even though the methyl acrylate/2-ethylhexyl acrylate copolymer is not blended to the base. For this reason, bases each containing 0.5% by mass or more (preferably 1% by mass or more) of polyvinyl alcohol need to be targeted for the case where a methyl acrylate/2-ethylhexyl acrylate copolymer is blended in the base for the purpose of inhibiting generation of an unpleasant odor originated from the polyvinyl alcohol. On the other hand, if the content of the polyvinyl alcohol exceeds 8% by mass, the adhesive force of the base becomes so strong that the patch causes intolerable pain at peeling-off. From the viewpoint of ensuring such an appropriate adhesive force as to cause only tolerable pain at peeling-off, the upper limit of the content of the polyvinyl alcohol is preferably 7.5% by mass or less.

The methyl acrylate/2-ethylhexyl acrylate copolymer used in the present invention is a copolymer of methyl acrylate with 2-ethylhexyl acrylate. The blending of this methyl acrylate/2-ethylhexyl acrylate copolymer enables inhibition of generation of the unpleasant odor originated from the gelatin and the polyvinyl alcohol. In the patch base of the present invention, the content of the methyl acrylate/2-ethylhexyl acrylate copolymer is 0.5 to 13% by mass relative to the total mass of the base. If the content of the methyl acrylate/2-ethylhexyl acrylate copolymer is less than 0.5% by mass, the unpleasant odor originated from the gelatin and the polyvinyl alcohol cannot be inhibited from being generated. On the other hand, if the content of the methyl acrylate/2-ethylhexyl acrylate copolymer exceeds 13% by mass, an odor of the methyl acrylate/2-ethylhexyl acrylate copolymer itself is generated. From the viewpoint of surely inhibiting generation of the unpleasant odor originated from the gelatin and the polyvinyl alcohol and the odor of the methyl acrylate/2-ethylhexyl acrylate copolymer itself, the content of the methyl acrylate/2-ethylhexyl acrylate copolymer is preferably 0.6 to 12% by mass, and more preferably 0.6 to 10% by mass.

In the present invention, it is preferable that the methyl acrylate/2-ethylhexyl acrylate copolymer be blended in an aqueous emulsion state in which the copolymer is dispersed in water during preparation of the patch base. In this case, the content of the methyl acrylate/2-ethylhexyl acrylate copolymer according to the present invention is the content of the methyl acrylate/2-ethylhexyl acrylate copolymer based on the solid content in the emulsion. The aqueous emulsion of the methyl acrylate/2-ethylhexyl acrylate copolymer preferably further contains a surfactant and/or a protective colloid agent in addition to the water and the methyl acrylate/2-ethylhexyl acrylate copolymer. The surfactant and/or the protective colloid agent is preferably poly(oxyethylene) nonyl phenyl ether. For this reason, the patch base of the present invention may further contain a surfactant and/or a protective colloid agent stemmed from the aqueous emulsion. When the surfactant and/or the protective colloid agent (preferably poly(oxyethylene) nonyl phenyl ether) is further contained, the total content thereof is preferably 0.01 to 0.5% by mass relative to the total mass of the base.

The patch base of the present invention contains L-menthyl glyceryl ether as a cooling agent. The L-menthyl glyceryl ether is not particularly limited, but may be any L-menthyl glyceryl ether generally used for patches. Since this L-menthyl glyceryl ether is odor-less, the patch base and the patch using the same of the present invention do not generate any irritating odor as generated by L-menthol. In the patch base of the present invention, the content of the L-menthyl glyceryl ether is preferably 0.5 to 7% by mass relative to the total mass of the base.

In addition, the patch base of the present invention preferably further contains at least one adhesive agent selected from the group consisting of polyacrylic acids and polyacrylic acid neutralized products. This adhesive agent improves the adhesive force of the base.

The polyacrylic acid is not particularly limited, but may be any polyacrylic acid generally used for patches. In the patch base of the present invention, the content of the polyacrylic acid is preferably 1 to 5% by mass relative to the total mass of the base. With this ingredient, the base tends to improve in the adhesive force and the adhesive layer tends to improve in the formability and shape retention. Further, the adhesive layer is also more unlikely to be too hard and therefore tends to improve in the adhesion to the skin.

The polyacrylic acid neutralized product is not particularly limited, but may be any polyacrylic acid neutralized product generally used for patches. Examples thereof include polyacrylates such as sodium salts, potassium salts, calcium salts, and ammonium salts. The polyacrylic acid neutralized product may be a partially neutralized product, a completely neutralized product, or a mixture of them. However, from the viewpoint that the adhesive force of the base further improves, a partially neutralized product of polyacrylic acid is preferable. The partially neutralized product of polyacrylic acid is a product in which structural units derived from the acrylic acid and structural units derived from the acrylate are present at a certain ratio in one polymer chain, and is preferably a product in which 20 mol% or more of carboxy groups in one polymer chain are neutralized. The content of the polyacrylic acid neutralized product is preferably 1 to 5% by mass relative to the total mass of the base. With this ingredient, the base tends to improve in the adhesive force and the adhesive layer tends to improve in the formability and shape retention.

Then, in the case where the patch of the present invention is a cataplasm, the patch base of the present invention contains water (purified water). The content of the water is preferably 20 to 95% by mass and more preferably 25 to 90% by mass relative to the total mass of the base. With this ingredient, the adhesive force exerted by the polyacrylic acid and the polyacrylic acid neutralized product is sufficiently ensured.

Moreover, the patch base of the present invention may further contain various additives such as solubilizing agents, humectants, stabilizers, inorganic powders, coloring agents, perfumes, and pH adjusters as long as the additives do not impair the effect of the present invention.

Next, the patch of the present invention is elaborated on. The patch of the present invention is a patch comprising an adhesive layer containing the base of the present invention and a drug on a backing.

The backing is not particularly limited, but may be any backing generally used for patches. Examples thereof include woven fabric (such as knitted fabric), nonwoven fabric, resin film, foamed sheet, and paper. Materials for the woven fabric, the nonwoven fabric, and the resin film are not particularly limited. Examples thereof include: polyolefins such as polyethylene, polypropylene, and polybutylene; polyesters such as polyethylene terephthalate; and rayon, polyurethane, and cotton.

In the patch of the present invention, an adhesive layer containing the base of the present invention and a drug (pharmacologically active substance) is formed on at least one of the surfaces of the backing. The drug is not particularly limited, but may be any drug generally used for patches. Examples thereof include nonsteroidal antiinflammatory agents such as felbinac, flurbiprofen, diclofenac, diclofenac sodium, methyl salicylate, glycol salicylate, indomethacin, and ketoprofen, or esters of them; antihistamines such as diphenhydramine; analgesics such as aspirin, acetaminophen, ibuprofen, and loxoprofen sodium; local anesthetics such as lidocaine; muscle relaxants such as suxamethonium chloride; antifungal agents such as clotrimazole; antihypertensive agents such as clonidine; vasodilators such as nitroglycerin and isosorbide nitrate; vitamins such as vitamin A, vitamin E (tocopherol), tocopherol acetate ester, vitamin K, octotiamine, and riboflavin butyrate ester; prostaglandins, scopolamine, fentanyl, capsicum extract, and nonanoic acid vanillylamide.

Moreover, the patch of the present invention may include a release liner on a surface of the adhesive layer opposite from the backing. This release liner covers and protects the adhesive layer. The release liner is not particularly limited but may be any release liner generally used for patches. Examples thereof include: resin films of polyesters (such as polyethylene terephthalate, polybutylene terephthalate, and polyethylene naphthalate), polyolefins (such as polyethylene, polypropylene, and polybutylene), and the like; paper sheets; and the like.

The method for manufacturing the patch of the present invention is not particularly limited, but any general patch manufacturing method may be employed. For example, the patch of the present invention may be obtained by preparing a paste containing the base of the present invention and a drug, uniformly spreading out the paste onto a release liner, and laminating a backing onto the spread paste.

### [Examples]

Hereinafter, the present invention will be described more specifically based on Examples and Comparative Examples; however, the present invention is not limited to the following Examples.

### (Test Example 1)

First, 1 to 5 parts by mass of gelatin was added to and dissolved in 99 to 95 parts by mass of purified water to prepare a sample solution in a total mass of 100 parts by mass. Each of these sample solutions was examined in a sensory test and the odor thereof was evaluated with an odor score within a range of 0 to 100 based on the following criteria. This sensory test involved evaluations by five subjects and the average value of the odor scores was calculated. The results are presented in Table 1.

### [Judgment Criteria]

- 100:: The odor was very unpleasant.
- 75:: The odor was unpleasant.
- 50:: The odor was noticeable but was not unpleasant.
- 25:: The odor was slightly noticeable.
- 0:: The odor was not noticeable at all.

### (Test Example 2)

The sensory test was conducted by the five subjects in the same way as in Test Example 1 except that polyvinyl alcohol (PVA) was used in place of the gelatin, and the average value of the odor scores was calculated. The results are presented in Table 1.

### (Test Example 3)

The sensory test was conducted by the five subjects in the same way as in Test Example 1 except that polyacrylic acid (PAA) was used in place of the gelatin, and the average value of the odor scores was calculated. The results are presented in Table 1.

**[Table 1]**

| Ingredient (parts by mass) | Test Example 1 | | | Test Example 2 | | | Test Example 3 | | |
|---|---|---|---|---|---|---|---|---|---|
| Gelatin | 1 | 2 | 5 | 0 | 0 | 0 | 0 | 0 | 0 |
| PVA | 0 | 0 | 0 | 1 | 2 | 5 | 0 | 0 | 0 |
| PAA | 0 | 0 | 0 | 0 | 0 | 0 | 1 | 2 | 5 |
| Purified water | 99 | 98 | 95 | 99 | 98 | 95 | 99 | 98 | 95 |
| Odor score | 33 | 54 | 67 | 71 | 79 | 93 | 0 | 0 | 0 |

As presented in Table 1, it was found that the gelatin and the polyvinyl alcohol were cause substances of the odor. In addition, it was found that when the contents of the gelatin and the polyvinyl alcohol were 2% by mass or more and 1% by mass or more, respectively, the odor was felt unpleasant.

### (Example 1)

First, a base was prepared by mixing and stirring 2 parts by mass of gelatin, 1 part by mass of polyvinyl alcohol (PVA), 0.6 parts by mass (solid content basis) of a methyl acrylate/2-ethylhexyl acrylate copolymer emulsion ("NIKASOL TS-620" manufactured by NIPPON CARBIDE INDUSTRIES CO., INC.), 1 part by mass of L-menthyl glyceryl ether, 1 part by mass of sodium polyacrylate (PANa), 0.1 parts by mass of magnesium silicate, and 94.3 parts by mass of purified water.

The obtained base was uniformly spread out onto a release liner such that an amount of the base per cataplasm (140 mm×100 mm) was 7 g. Immediately after that, a backing was laminated onto the base to obtain a cataplasm.

### (Examples 2 to 16)

Each base was prepared in the same way as in Example 1 except that the amounts of the gelatin, the polyvinyl alcohol (PVA), the methyl acrylate/2-ethylhexyl acrylate copolymer emulsion, and the purified water blended were changed to the amounts presented in Table 2, and then a cataplasm was produced.

### (Comparative Example 1)

A base was prepared in the same way as in Example 4 except that 12 parts by mass of an aminoalkyl methacrylate copolymer E ("EUDRAGIT EPO" manufactured by Evonik Industries AG) was used in place of the methyl acrylate/2-ethylhexyl acrylate copolymer emulsion, and then a cataplasm was produced.

### (Comparative Example 2)

A base was prepared in the same way as in Example 4 except that 12 parts by mass of a butyl methacrylate/methyl methacrylate copolymer ("Plastoid B" manufactured by Evonik Industries AG) was used in place of the methyl acrylate/2-ethylhexyl acrylate copolymer emulsion, and then a cataplasm was produced.

### (Comparative Examples 3 to 13)

Each base was prepared in the same way as in Example 1 except that the amounts of the gelatin, the polyvinyl alcohol (PVA), the methyl acrylate/2-ethylhexyl acrylate copolymer emulsion, and the purified water blended were changed to the amounts presented in Table 3, and then a cataplasm was produced.

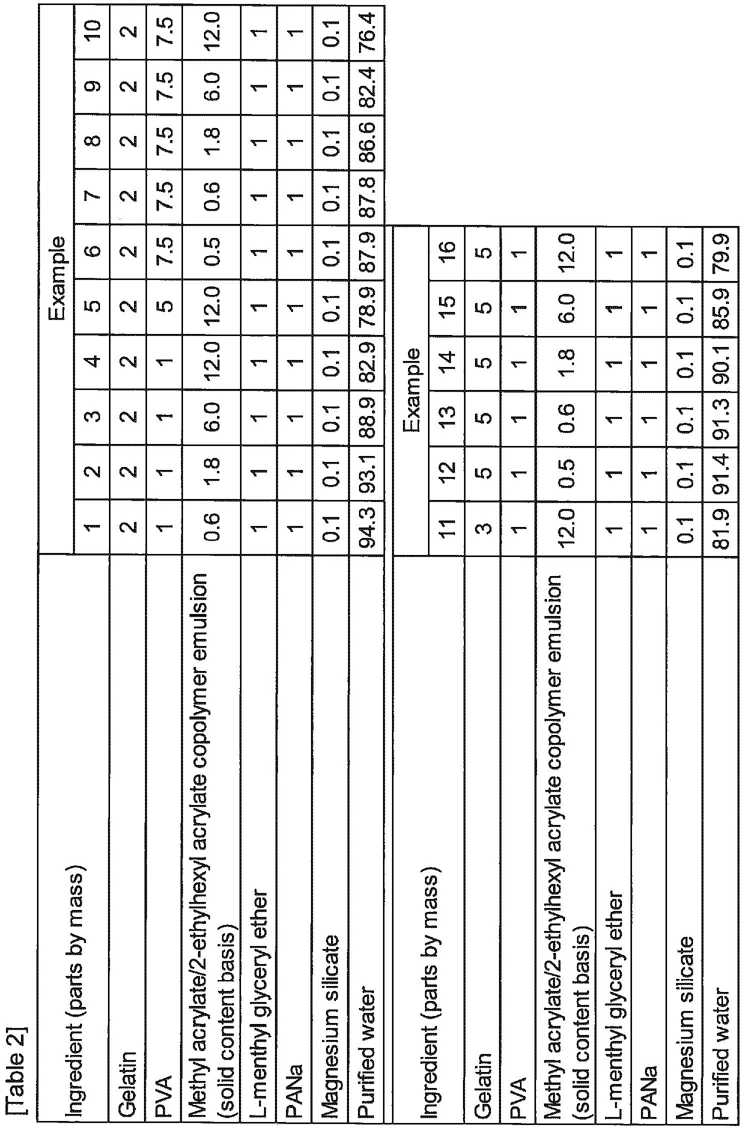

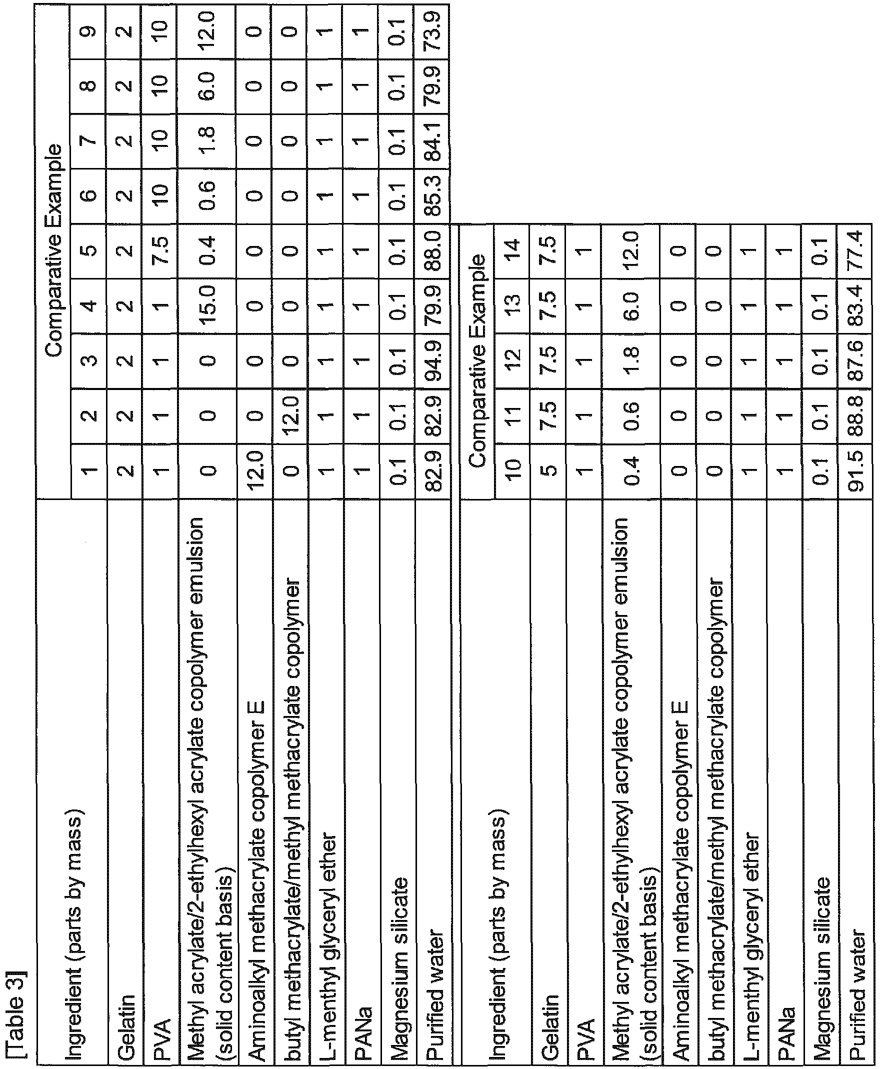

### <Tackiness Test>

The obtained cataplasms each were cut into a rectangle of 25 mm × 90 mm, and placed on a horizontal bottom surface under a sinusoidal-curved inclined stage. The release liner was removed to expose the base layer. Next, a No. 17 steel ball (diameter: 17/32 inches) was rolled on the inclined surface of the inclined stage, and a distance (stop distance) by which the steel ball moved on the surface of the base layer until it stopped was measured. This measurement was repeated three times, and the average value of them (unit: mm) was calculated. Tables 4 to 7 present the results. Here, the tackiness was expressed by "90<" if the steel ball did not stop on the surface of the base layer. As the stop distance becomes shorter, the adhesive force of the base layer is judged to be higher. The adhesive force is acceptable as a formulation if the stop distance is 90 mm or shorter. However, if the stop distance is too short, the adhesive force is too strong. In this case, the patch causes intolerable pain at peeling-off. For this reason, the stop distance is preferably 20 mm or longer.

### <Sensory Test>

The obtained cataplasms each were examined in a sensory test, and evaluated as an odor score within a range of 0 to 100 based on the following judgment criteria. This sensory test involved evaluations by five subjects and the average value of the odor scores was calculated. The results are presented in Tables 4 to 7. Here, the sensory test was not conducted on the cataplasm, of which the result of the tackiness test was "90<" or shorter than 20 mm.

### [Judgment Criteria]

- 100:: The odor was very unpleasant.
- 75:: The odor was unpleasant.
- 50:: The odor was noticeable but was not unpleasant.
- 25:: The odor was slightly noticeable.
- 0:: The odor was not noticeable at all.

**[Table 4]**

| Ingredient (parts by mass) | Example | Comp. Example | |
|---|---|---|---|
| | 4 | 1 | 2 |
| Gelatin | 2 | 2 | 2 |
| PVA | 1 | 1 | 1 |
| Methyl acrylate/2-ethylhexyl acrylate copolymer emulsion (solid content basis) | 12.0 | 0 | 0 |
| Aminoalkyl methacrylate copolymer E | 0 | 12.0 | 0 |
| butyl methacrylate/methyl methacrylate copolymer | 0 | 0 | 12.0 |
| Odor score | 29 | 63 | 71 |

As is apparent from the results in Table 4, it was observed that the use of the methyl acrylate/2-ethylhexyl acrylate copolymer emulsion (Example 4) enabled masking of the formulation odor originated from the gelatin and the PVA (the odor scores of 50 or less). On the other hand, the use of the aminoalkyl methacrylate copolymer E (Comparative Example 1) or the butyl methacrylate/methyl methacrylate copolymer (Comparative Example 2) in place of the methyl acrylate/2-ethylhexyl acrylate copolymer emulsion resulted in a failure in masking of the formulation odor (the odor scores of more than 50).

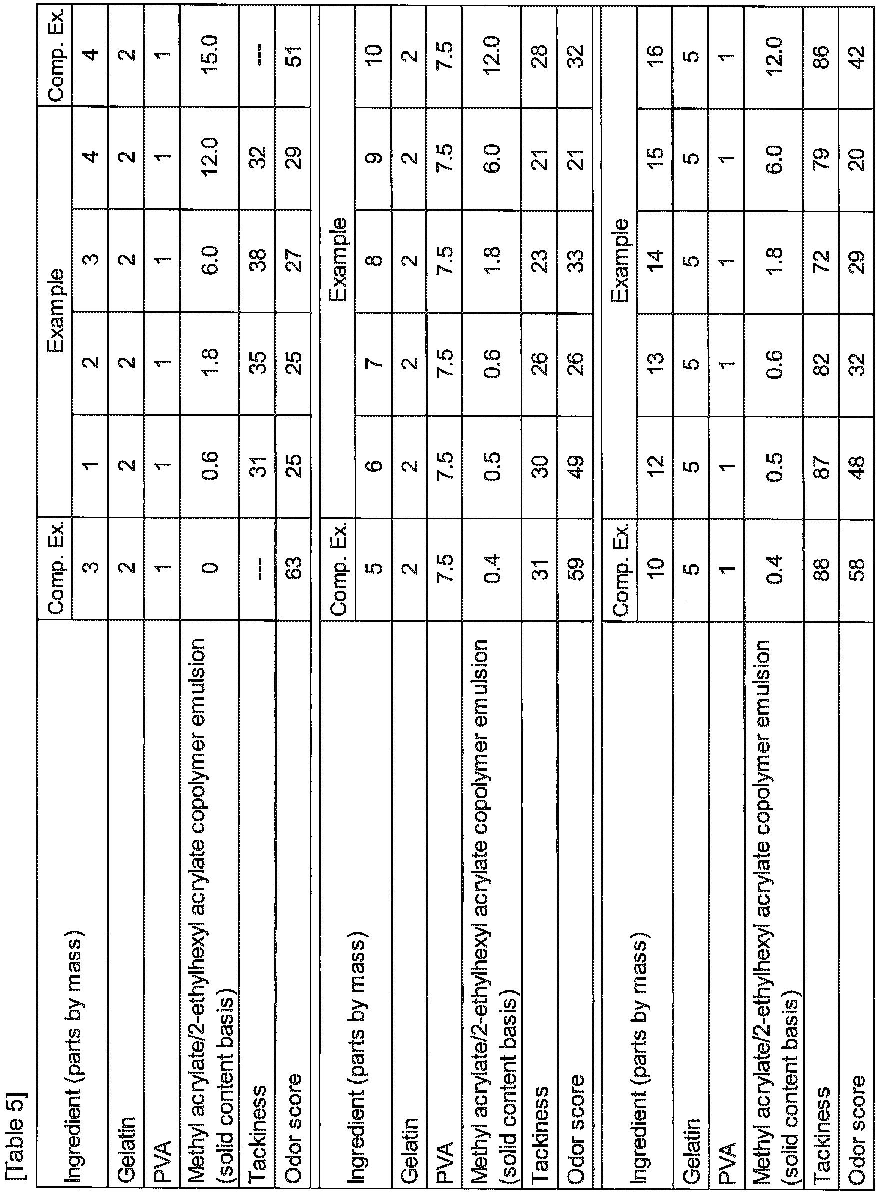

As is apparent from the results in Table 5, it was observed that the methyl acrylate/2-ethylhexyl acrylate copolymer emulsion added in a content (solid content basis) within a range of 0.5 to 13.0% by mass (Examples 1 to 4, 6 to 10, and 12 to 16) relative to the total mass of the base enabled masking of the formulation odor originated from the gelatin and the PVA (the odor scores of 50 or less), and achieved the adhesive force acceptable as a formulation (the stop distances of 20 to 90 mm). On the other hand, the methyl acrylate/2-ethylhexyl acrylate copolymer emulsion added in a content (solid content basis) of less than 0.5% by mass (Comparative Examples 3, 5, and 10) resulted in a failure in masking of the formulation odor (the odor scores of more than 50). Meanwhile, it was found that the methyl acrylate/2-ethylhexyl acrylate copolymer emulsion (solid content basis) added in a content of 15.0% by mass (Comparative Example 4) resulted in generation of an odor of the methyl acrylate/2-ethylhexyl acrylate copolymer itself (the odor score of more than 50).

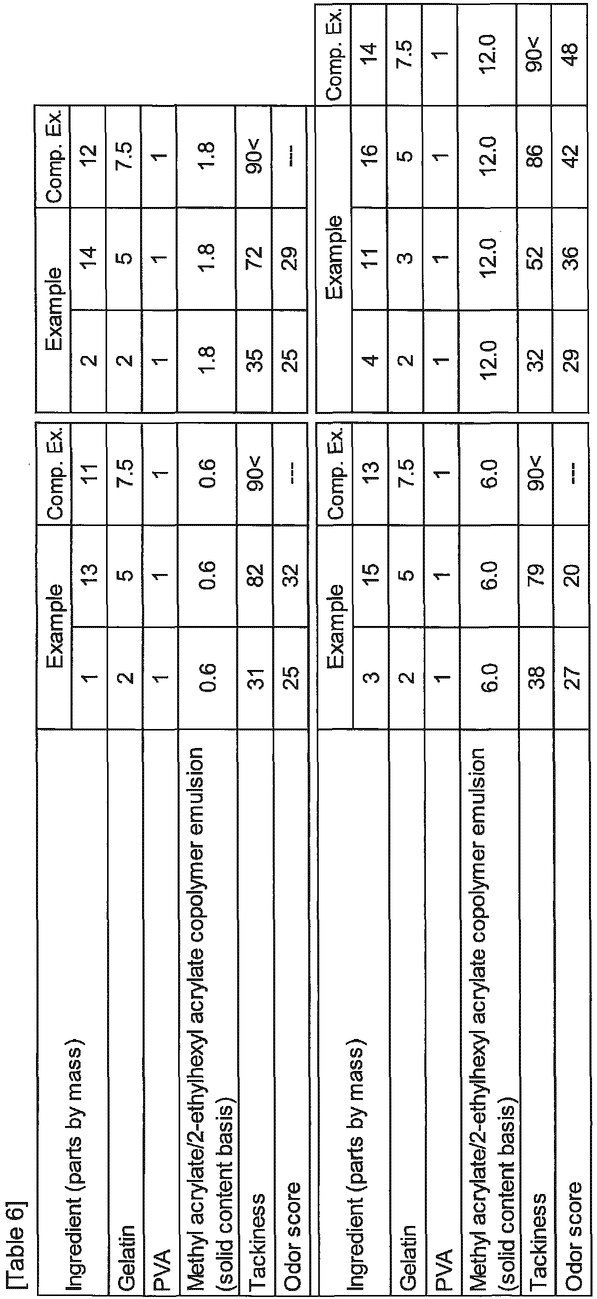

As is apparent from the results in Table 6, it was observed that the base in which the content of the methyl acrylate/2-ethylhexyl acrylate copolymer emulsion (solid content basis) was 0.5 to 13.0% by mass enabled masking of the formulation odor originated from the gelatin (the odor scores of 50 or less) and also achieved the adhesive force acceptable as a formulation (the stop distances of 20 to 90 mm), when the content of the gelatin was within a range of 1.5 to 6% by mass (Examples 1 to 4, 11, and 13 to 16) relative to the total mass of the base. On the other hand, the content of the gelatin of 7.5% by mass (Comparative Examples 11 to 14) resulted in too weak an adhesive force (the stop distances of longer than 90 mm), which is not acceptable as formulations.

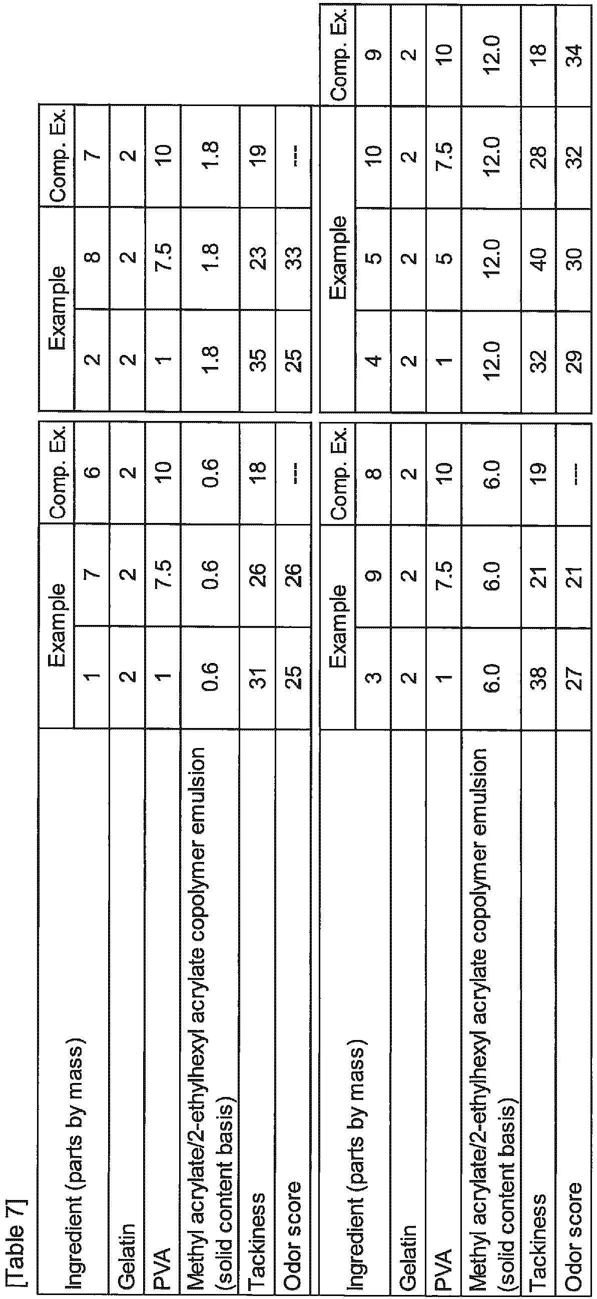

As is apparent from the results in Table 7, it was observed that the base in which the content of the methyl acrylate/2-ethylhexyl acrylate copolymer emulsion (solid content basis) was 0.5 to 13.0% by mass enabled masking of the formulation odor originated from the PVA (the odor scores of 50 or less) and also achieved the adhesive force acceptable as a formulation (the stop distances of 20 to 90 mm), when the content of the polyvinyl alcohol (PVA) was within a range of 0.5 to 8% by mass (Examples 1 to 5 and 7 to 10) relative to the total mass of the base. On the other hand, the content of the PVA of 10% by mass (Comparative Examples 6 to 9) resulted in too strong an adhesive force (the stop distances of shorter than 20 mm), which seems to cause intolerable pain at peeling-off.

### (Example 17)

First, a paste containing a base and a drug was prepared by mixing and stirring 2 parts by mass of gelatin, 5 parts by mass of polyvinyl alcohol (PVA), 10 parts by mass (solid content basis) of a methyl acrylate/2-ethylhexyl acrylate copolymer emulsion ("NIKASOL TS-620" manufactured by NIPPON CARBIDE INDUSTRIES CO., INC.), 1 part by mass of L-menthyl glyceryl ether, 1 part by mass of sodium polyacrylate (PANa), 0.1 parts by mass of magnesium silicate, 2 parts by mass of glycol salicylate, 1 part by mass of tocopherol acetate ester, 1 part by mass of polyethylene glycol monostearate, and 76.9 parts by mass of purified water.

The obtained paste was uniformly spread out onto a release liner such that an amount of the paste per cataplasm (140 mm×100 mm) was 7 g. Immediately after that, a backing was laminated onto the paste to obtain a cataplasm. This cataplasm was examined in the above-specified tackiness test and sensory test. The results are presented in Table 8.

**[Table 8]**

| Ingredient (parts by mass) | Example 17 |
|---|---|
| Gelatin | 2 |
| PVA | 5 |
| Methyl acrylate/2-ethylhexyl acrylate copolymer emulsion (solid content basis) | 10 |
| L-menthyl glyceryl ether | 1 |
| PANa | 1 |
| Magnesium silicate | 0.1 |
| Glycol salicylate | 2 |
| Tocopherol acetate ester | 1 |
| Polyethylene glycol monostearate | 1 |
| Purified water | 76.9 |
| Tackiness | 30 |
| Odor score | 10 |

As is apparent from the results in Table 8, it was observed that even in the case where the cataplasm using L-menthyl glyceryl ether as a cooling agent contains a drug (in other words, the cataplasm comprises the adhesive layer containing the base and the drug), the blending of the poly(methyl acrylate/2-ethylhexyl acrylate) copolymer into the adhesive layer resulted in inhibition of generation of the unpleasant odor generated from the gelatin and the polyvinyl alcohol while retaining the adhesive force of the adhesive layer.

### [Industrial Applicability]

According to the present invention, it is possible to provide a patch base which retains an adhesive force of the base and inhibits generation of an unpleasant odor even in the case of using L-menthyl glyceryl ether as a cooling agent.

Therefore, since the adhesive layer is formed by using the aforementioned base, the patch of the present invention is advantageously useful in that one can use the patch for a long term without feeling an unpleasant odor such as an irritating odor and a formulation odor.

## Claims

1. A patch base comprising gelatin, polyvinyl alcohol, a methyl acrylate/2-ethylhexyl acrylate copolymer, and L-menthyl glyceryl ether, wherein
relative to the total mass of the base,
the content of the gelatin is 1.5 to 6% by mass,
the content of the polyvinyl alcohol is 0.5 to 8% by mass, and
the content of the methyl acrylate/2-ethylhexyl acrylate copolymer is 0.5 to 13% by mass.

2. The patch base according to claim 1, further comprising at least one adhesive agent selected from the group consisting of polyacrylic acids and polyacrylic acid neutralized products.

3. A patch comprising an adhesive layer containing the base according to claim 1 or 2 and a drug on a backing.

## Patentansprüche

1. Pflasterbasis, umfassend Gelatine, Polyvinylalkohol, ein Methylacrylat/2-Ethylhexylacrylat-Copolymer und L-Menthylglycerylether, wobei
bezogen auf die Gesamtmasse der Basis
der Gehalt der Gelatine 1,5 bis 6 Masse-% beträgt,
der Gehalt des Polyvinylalkohols 0,5 bis 8 Masse-% beträgt, und
der Gehalt des Methylacrylat/2-Ethylhexylacrylat-Copolymers 0,5 bis 13 Masse-% beträgt.

2. Pflasterbasis nach Anspruch 1, ferner umfassend mindestens ein Klebemittel, ausgewählt aus der Gruppe bestehend aus Polyacrylsäuren und neutralisierten Produkten von Polyacrylsäure.

3. Pflaster, umfassend eine Klebeschicht, enthaltend die Basis nach Anspruch 1 oder 2 und einen Wirkstoff auf einem Träger.

## Revendications

1. Base pour timbre comprenant de la gélatine, du poly(alcool de vinyle), un copolymère d'acrylate de méthyle/acrylate de 2-éthylhexyle, et de l'éther de *L-*menthyl glycéryle,
par rapport à la masse totale de la base,
la teneur de la gélatine étant de 1,5 à 6 % en masse,
la teneur du poly(alcool de vinyle) étant de 0,5 à 8 % en masse, et
la teneur du copolymère d'acrylate de méthyle/acrylate de 2-éthylhexyle étant de 0,5 à 13 % en masse.

2. Base pour timbre selon la revendication 1, comprenant en outre au moins un agent adhésif sélectionné dans le groupe constitué des poly(acides acryliques) et des produits neutralisés de poly(acide acrylique).

3. Timbre comprenant une couche adhésive contenant la base selon la revendication 1 ou 2 et un médicament sur une partie dorsale.
